# EUROPÄISCHE PATENTSCHRIFT

(11) **EP 2 691 022 B1**
(45) Veröffentlichungstag und Bekanntmachung des Hinweises auf die Patenterteilung: **07.01.2015**
(21) Anmeldenummer: 12709848.1
(22) Anmeldetag: 15.03.2012
(51) Int. Cl.: A61B 5/15, A61B 5/151

(54) **STECHVORRICHTUNG MIT DREHELEMENT**
PIERCING DEVICE WITH ROTARY ELEMENT
DISPOSITIF PIQUANT À ÉLÉMENT ROTATIF

(30) Priorität: 31.03.2011 DE 102011015758
(43) Veröffentlichungstag der Anmeldung: 05.02.2014
(73) Patentinhaber: Gerresheimer Regensburg GmbH, 93047 Regensburg (DE)
(72) Erfinder: BUTZ, Marion, 93049 Regensburg (DE); STREHL, Michael-Martin, 92536 Pfreimd (DE)
(74) Vertreter: Hannke, Christian
(86) Internationale Anmeldenummer: PCT/EP2012/054519
(87) Internationale Veröffentlichungsnummer: WO 2012/130619

(56) Entgegenhaltungen:
- EP-A2- 1 090 584
- WO-A1-2008/107382
- WO-A1-2009/147080
- US-A- 5 545 174
- US-A1- 2005 145 520

## Beschreibung

Die Erfindung betrifft eine Stechvorrichtung für die Blutentnahme bei medizinischen Untersuchungen mit einem Basiskörper, mit mindestens einer darin angeordneten und mit einem Spitzende herausfahrbaren Nadel, mit einem die Nadel zumindest teilweise umfassenden Nadelhalteelement und einer Antriebseinheit zum Antreiben einer Bewegung der Nadel zusammen mit dem Nadelhalteelement gegenüber dem Basiskörper.

Derartige Stechvorrichtungen sind in vielfältiger Weise bekannt. Beispielsweise gibt es Stechvorrichtungen, die mittels einer Antriebseinheit ein stößelartiges Element mit einer damit verbundenen Feder in verschiedenster Form, wie beispielsweise eine Spiralfeder oder einer Schenkelfeder, und ein Nadelhalteelement aufweisen, welches durch diesen Stößel angetrieben wird. Hierdurch wird die Nadel bzw. die Lanzette nach vorne geschoben und tritt aus der Stechvorrichtung bzw. den Basiskörper aus, um in die Haut eines Patienten einzustechen.

Sofern gewünscht ist, dass die Lanzette bzw. die Nadel unmittelbar nach dem Stechvorgang wieder zurückfährt und in den Basiskörper bzw. das Gehäuse der Stechvorrichtung verschwindet, gibt es bei derartigen Stechvorrichtungen verschiedenste Arten von Kurvenbahnen, die aufgrund des Kurvenverlaufs das Nadelhalteelement, welches beispielsweise mit einem Vorsprung, der innerhalb einer derartigen Kurvenbahn verläuft, ausgestattet ist, zunächst nach vorne bzw. aus den Basiskörper heraustreten lassen und anschließend wieder in den Körper hineinfahren lassen, da die Kurvenbahn an dieser Stelle wieder rückläufig bzw. nach hinten verlaufend ausgebildet ist.

Derartige Stechvorrichtungen weisen im Allgemeinen den Nachteil auf, dass sie eine zusätzliche Bewegung der Nadel bzw. des Nadelhalteelementes erfahren, wenn die Stechvorrichtung erneut vorgespannt wird, nachdem zuvor ein Stechvorgang durchgeführt worden ist und bevor ein weiterer Stechvorgang ausgelöst wird. Die hierdurch bedingte Bewegung der Lanzette bzw. der Nadel ist in der Regel nicht gewünscht, da hierdurch eine hohe Komplexität ineinander wirkender Bauteile, ein großer Bauraum, hohe Herstellungskosten und die Bewegung von vielen Bauteilen während des Spannvorganges bedingt ist.

Demzufolge ist es Aufgabe der Erfindung, eine Stechvorrichtung für die Blutentnahme bei medizinischen Untersuchungen mit einem Basiskörper, einer Nadel, einem Nadelhalteelement und einer Antriebseinheit zur Verfügung zu stellen, bei der ein Vorspannvorgang für einen erneuten Stechablauf bzw. Stechvorgang ohne die gleichzeitige Bewegung der Nadel stattfinden kann.

Diese Aufgabe wird gemäß den Merkmalen des Patentanspruches 1 gelöst.

Ein wesentlicher Punkt der Erfindung liegt darin, dass bei einer Stechvorrichtung für die Blutentnahme bei medizinischen Untersuchungen mit einem Basiskörper, mindestens einer darin angeordneten und mit einem Spitzende herausfahrbaren Nadel, mit einem die Nadel zumindest teilweise umfassenden Nadelhalteelement und einer Antriebseinheit zum Antreiben einer Bewegung der Nadel zusammen mit dem Nadelhalteelement gegenüber dem Basiskörper ein Drehelement angeordnet ist, welches zur Durchführung einer Drehbewegung zwischen der Antriebseinheit und dem Nadelhalteelement als Verbindungselement vorgesehen ist und die Drehbewegung sowohl links- als auch rechtsgerichtet jeweils die Bewegung des Nadelhalteelementes sowohl in Vorwärts- als auch im Rückwärtsrichtung bewirkt.

Mit anderen Worten kann durch die Anordnung eines derartigen Drehelementes unabhängig davon, ob sich das Drehelement nach links dreht oder nach rechts dreht, also mit dem Uhrzeigersinn oder gegen den Uhrzeigesinn, während einer derartigen Drehbewegung in ausschließlich eine Richtung sowohl eine vorwärts- als auch eine rückwärtsgerichtete Bewegung des Nadelhalteelementes bewirkt werden.

Das Antriebselement ist linear beweglich und mit mindestens zwei Eingreifarmen ausgestattet, wovon bei jeder Drehbewegung jeweils einer der Eingreifarme in einen von mindestens zwei Vorsprüngen des Drehelementes eingreift und der andere Vorsprung mittels einer Kurvenbahn an dem anderen Eingreifarm vorbeilenkbar ist, ohne dass er mit dem anderen Eingreifarm eine Eingreifposition einnimmt. Hierdurch wird ermöglicht, dass abwechselnd in Abhängigkeit von der Drehrichtung der Drehbewegung entweder der eine Eingreifarm oder der andere Eingreifarm in einen der Vorsprünge eingreift, jedoch zugleich der weitere Eingreifarm in den verbleibenden Vorsprung nicht eingreift. Dies ermöglicht eine Art Hin- und Herdrehen des Drehelementes für die verschiedenen Stechvorgänge. Hierbei ist wichtig, dass der erste Vorsprung und der erste Eingreifarm, in Stechrichtung betrachtet, links von einer Längsachse, die durch eine Drehachse des Drehelementes verläuft, und der zweite Vorsprung sowie der zweite Eingreifarm rechts von der Längsachse angeordnet sind. Auf diese Weise kann immer über die Längsachse hinweg von links nach rechts oder von rechts nach links eine Drehbewegung des Vorsprunges, der in den Eingreifarm momentan eingreift, bewirkt werden und somit auch ein Nachvorne- und -hintenver-schieben des Nadelhalteelementes, welches mit dem Drehelement ebenso verbunden ist.

Es wird somit eine Art Kurvenbahn durch die Drehbewegung des Drehelementes beschrieben und zugleich durch die Anordnung eines Eingreifarmes und der zwei Vorsprünge auf dem Drehelement sichergestellt, dass die Lanzette bzw. die Nadel sich nach vorne und nach hinten bewegt, sowohl bei einer linksgerichteten als auch bei einer rechtsgerichteten Drehbewegung. Hieraus entfällt die Notwendigkeit, die Lanzette bzw. die Nadel nochmals zu bewegen, wenn ein Vorspannvorgang stattfindet, um das Antriebselement erneut für einen neuen Stechvorgang vorzuspannen.

Vorteilhaft ist das Drehelement als scheibenartiges Element mit der senkrecht zur Scheibenebene und zur Richtung der Bewegung des Nadelhalteelementes angeordneten Drehachse ausgebildet und die beiden Vorsprünge sind auf biegsam ausgebildeten, zungenartigen Elementen der Scheibenebene randseitig angeordnet, wobei die zungenartigen Elemente in Richtung des Verlaufs der Drehachse auslenkbar sind. Dies hat zur Folge, dass aufgrund der Auslenkung von einem der beiden zungenartigen Elemente ein Vorbeilenken des darauf angeordneten Vorsprungs gegenüber dem ihm zugeordneten Eingreifarm möglich ist, während der andere Engreifarm in den Vorsprung eingreift und den eigentlichen Stechvorgang hierdurch durchführt.

Ein erster Abschnitt der Kurvenbahn ist unterhalb eines ersten zungenartigen Elementes und ein zweiter Abschnitt ist unterhalb eines zweiten zungenartigen Elementes mit einer Steigung zu dem scheibenartigen Element angeordnet. Eine derartige rampenartige Ausbildung der Kurvenbahn bewirkt, dass der eine Vorsprung abgesenkt werden kann und zwar mithilfe einer Vorspannung, die dem zungenartigen Element zueigen ist oder mithilfe eines zapfenartigen Elementes, welches unter dem zungenartigen Element angeordnet ist und in einer den Zapfen umgebenden Bahn, die die rampenartige Kurvenbahn in ihren Verlauf einnimmt und den Zapfen umgreift sowie nach unten zieht.

Die Abschnitte der Kurvenbahnen sind vorzugsweise kreisbogenförmig ausgebildet. Dies trifft im Übrigen auch vorzugsweise auf die zungenartigen Elemente zu.

Gemäß einer bevorzugten Ausführungsform ist mindestens ein Pleuelelement angeordnet, welches das Drehelement mit dem Nadelhalteelement gelenkig verbindet, wobei ein erstes Ende des Pleuelelementes mit einem Vorsprung in ein am Drehelement angeordnetes Langloch eingreift und ein zweites Ende des Pleuelelementes mit einem Vorsprung in eine komplementär dazu ausgebildete Ausnehmung des Nadelhalteelementes eingreift. Dies ermöglicht, dass die Drehbewegung des Drehelementes in eine translatorische bzw. lineare Bewegung des Nadelhalteelementes übertragen werden kann, unabhängig davon, ob das Drehelement sich nach links oder nach rechts dreht.

Das erste Ende des Pleuelelementes greift ebenso in eine mit dem Basiskörper verbundene weitere Kurvenbahn ein, deren Verlauf V-förmig und/oder kurvenförmig mit einem mittig angeordneten Maximum in Richtung des Nadelhalteelementes ausgestattet ist. Dies stellt sicher, dass das Pleuelelement ebenso mittels der weiteren Kurvenbahn auf einen vorbestimmten Weg geführt wird, wobei diese Kurvenbahn ohnehin bereits durch die Befestigung des Pleuelelementes an dem Drehelement sichergestellt ist. Insofern dient eine derartige weitere Kurvenbahn als Gegenlagerung für das Ende des Pleuelelementes.

Vorzugsweise ist die Antriebseinheit mit mindestens einem Federelement federbeaufschlagbar, wobei es sich hierbei um eine in Stechrichtung wirkende Spiralfeder handeln kann, die innerhalb der Antriebseinheit angeordnet ist und ein stößelartiges Element mit daran angeordneten Eingreifarmen nach vorne bewegen oder nach hinten bewegen kann.

Während einer Drehbewegung des Drehelementes ist jeweils ein Vorsprung, von dem darin eingreifenden Eingreifarm auf einer Kreisbogenbahn in einer Ebene von der einen Seite auf die andere Seite der Längsachse bewegbar.

Weitere vorteilhafte Ausführungsformen ergeben sich aus den Unteransprüchen.

Vorteile und Zweckmäßigkeiten sind der nachfolgenden Beschreibung in Verbindung mit der Zeichnung zu entnehmen. Hierbei zeigen:
- Fig. 1: In einer perspektivischen Darstellung Teile einer erfindungsgemäßen Stechvorrichtung;
- Fig. 2: In einer perspektivischen Darstellung Teile der erfindungsgemäßen Stechvorrichtung mit sichtbarem Federelement;
- Fig. 3: in einer Explosionsdarstellung die Teile der erfindungsgemäßen Stechvorrichtung;
- Fig. 4: in einer Ausschnittsdarstellung ein Teil der in Fig. 1 - 3 gezeigten Stechvorrichtung in einer Draufsicht;
- Fig. 5: in einer Seitendarstellung ein Ausschnitt der in Fig. 1 - 3 gezeigten Teile der erfindungsgemäßen Stechvorrichtung;
- Fig. 6: in einer perspektivischen Darstellung ein Ausschnitt in Fig. 1 - 3 gezeigten Teile der erfindungsgemäßen Stechvorrichtung;
- Fig. 7: in einer perspektivischen Darstellung ein Drehelement der erfindungsgemäßen Stechvorrichtung;
- Fig. 8: in einer Seitendarstellung das Drehelement mit einer Kurvenbahn gemäß einer ersten Ausführungsform der Erfindung;
- Fig. 9: das Drehelement mit einer der erfindungsgemäßen Stechvorrichtung mit einer Kurvenbahn gemäß einer zweiten Ausführungsform der Erfindung; und
- Fig. 10: in einer Draufsicht das Drehelement mit weiteren Bauteilen der erfindungsgemäßen Stechvorrichtung.

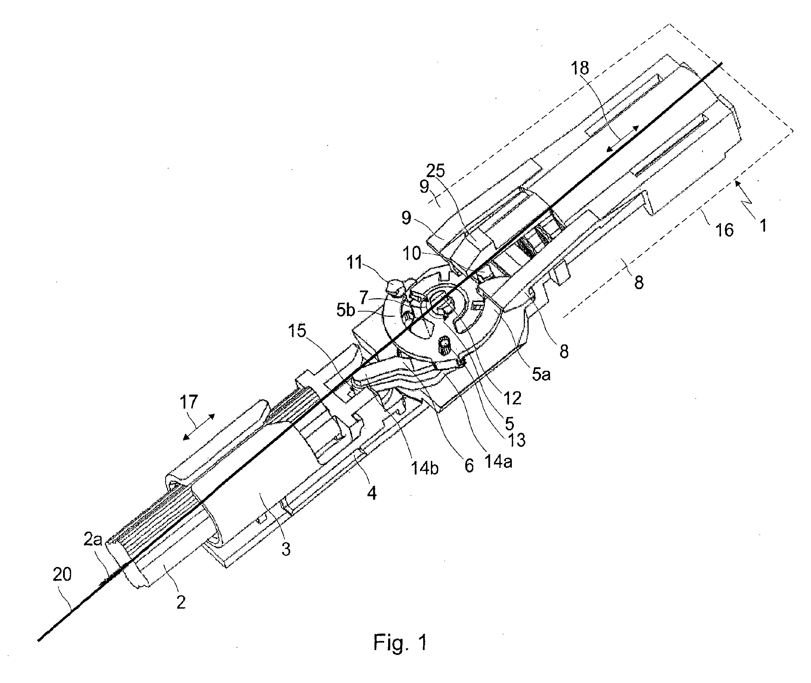

In Fig. 1 ist in einer perspektivischen Darstellung die erfindungsgemäße Stechvorrichtung in Teilen wiedergegeben. Im Wesentlichen besteht die erfindungsgemäße Stechvorrichtung aus den nachfolgenden im Inneren eines Gehäuses 16, welches hier lediglich angedeutet ist, angeordneten Teilen:

Eine Antriebseinheit 1, eine Nadel 2 mit einem Nadelhalteelement 3, ein Führungselement 4, in dem Nadelhalteelement entlang einer Richtung 17 linear verschiebbar ist, ein Drehelement 5 und eine Pleuelstange 6.

Die Antriebseinheit 1 kann gemäß dem Doppelpfeil 18 in Vorwärts- und Rückwärtsrichtung verschoben werden, sodass hierdurch die Nadel 2 mit ihrem Spitzende 2a aus einem hier nicht näher dargestellten Gehäuse ausfahren und wieder einfahren kann. Dies wird durch den Doppelpfeil 17 wiedergegeben.

In Verlängerung zu der Nadel bzw. der Lanzette 2 ist das Drehelement 5 mit einer Drehachse 12 angeordnet, welche auf einer Längsachse 20 liegt. Das Drehelement 5 kann gemäß dem Doppelpfeil 7 um diese Achse 12 gedreht werden, wobei die Achse 12 senkrecht zu der Längsachse 20 und senkrecht zu der vorwärts- und rückwärtsgerichteten Bewegung 17 der Nadel 2 ausgerichtet ist.

Das Drehelement 5 weist zwei zungenartige Elemente 5a und 5b auf, die sich entweder selbständig nach unten auslenken beziehungsweise verbiegen lassen aufgrund einer vorgearbeiteten Vorspannungskraft, sobald kein Gegenelement von unten mehr dagegen drückt oder mittels darunter angeordneten Kulissenbahnen beziehungsweise Kurvenbahnen mit an den zungenartigen Elementen 5a und 5b angeordneten Zapfen nach unten gezogen werden, sobald kein Gegenlager beziehungsweise ein gegenbeaufschlagtes Element von unten dagegen wirkt.

Das Drehelement 5 ist drehscheibenartig ausgebildet und stellt eine Verbindung zwischen der Antriebseinheit 1, die in Vorwärts- und Rückwärtsrichtung bewegt werden kann, und der Pleuelstange 6, die wiederum mit dem Nadelhalteelement 3 verbunden ist, dar.

Die Pleuelstange 6 ist mittels eines Zapfens 13 mit dem Drehelement 5 verbunden. Dieser Zapfen ist an einem ersten Ende 14a der Pleulstange 6 angeordnet.

An dem zweiten Ende 14b der Pleuelstange 6 ist ebenso ein Zapfen 15 angeordnet, der wiederum mit dem Nadelhalteelement 3 verbunden ist, sodass bei einer Drehung des Drehelementes 5 eine vorwärts und rückwärts gerichtete Bewegung 17 des Nadelhalteelementes 13 aufgrund des Gleitens innerhalb des Führungselementes 4 stattfindet.

Zwei Vorsprünge 10, 11, die oberseitig auf dem Drehelement 5 angeordnet sind, können in zwei Eingreifarme 8, 9 der Antriebseinheit 1 eingreifen, wobei immer nur jeweils einer dieser Vorsprünge 10, 11 in einen der Eingreifarme 8, 9 eingreift.

Ein weiterer Eingreifarm 25 dient dazu, die Antriebseinheit 1 nach erfolgtem Stechvorgang mittels weiterer hier nicht näher dargestellter Bauteile, die an dem Gehäuse 16 angeordnet sind, und mit einem Handbetätigungshebel verbunden sein können, wieder vorzuspannen.

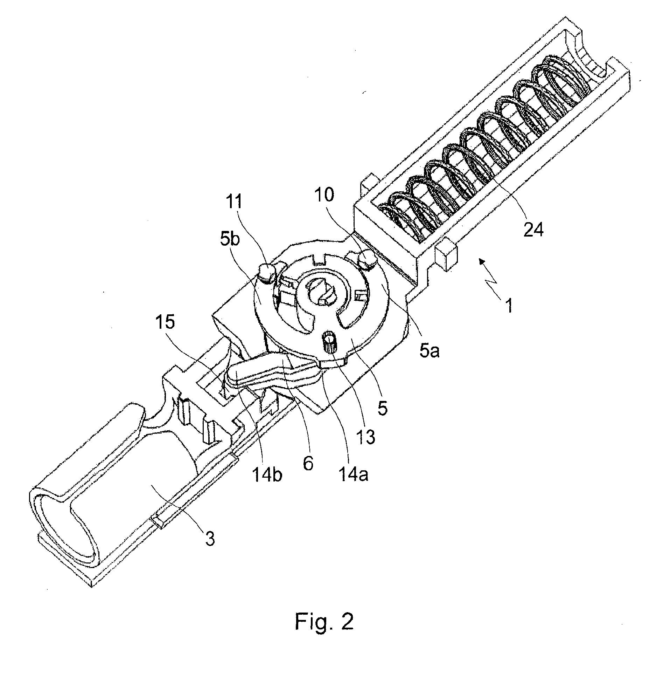

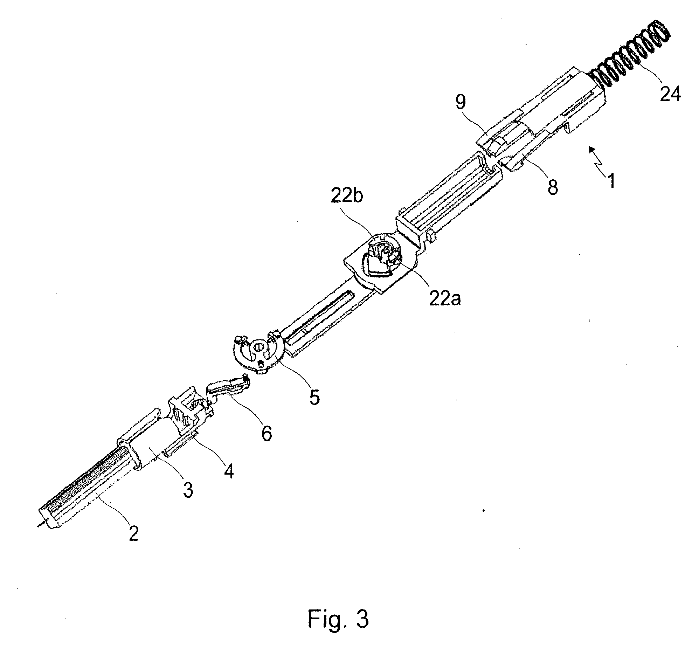

In Fig. 2 und in Fig. 3 ist die in Fig. 1 dargestellte erfindungsgemäße Stechvorrichtung nochmals mit weiteren Elementen oder in einer Explosionsdarstellung wiedergegeben.

In Fig. 2 ist ein Federelement 24 innerhalb der Antriebseinheit 1 zu sehen, welches für die Einstellung einer Vorspannung, um anschließend einen Stechvorgang durchführen zu können, zuständig ist. Dieses Federelement in Form einer Spiralfeder, die in Längsrichtung gemäß der Längsachse 20 der Stechvorrichtung wirken kann, kann im auseinandergezogenen Zustand vorgespannt vorliegen und zieht anschließend mittels hier nicht näher dargestellter Bauteile die Eingreifarme 8, 9 nach hinten, um das Drehelement 5 zu einer Drehung 7 zu veranlassen. In dieser Darstellung ist die Lanzette beziehungsweise Nadel 2 nicht dargestellt, vielmehr ist lediglich das Nadelhalteelement 3 dargestellt.

In Fig. 3 sind in einer Explosionsdarstellung nochmals die Einzelteile der in Fig. 1 und Fig. 2 dargestellten erfindungsgemäßen Stechvorrichtung wiedergegeben. Dieser Darstellung ist zu entnehmen, dass die erfindungsgemäße Stechvorrichtung im wesentlichen aus der Nadel 2, dem Nadelhalteelement 3, dem Führungselement 4, der Pleuelstange 6, dem Drehelement 5, Kulissenbahnabschnitten 22a und 22b, den Eingreifarmen 8, 9 mit der dazugehörigen Antriebseinheit 1 sowie der Spiralfeder 24 besteht.

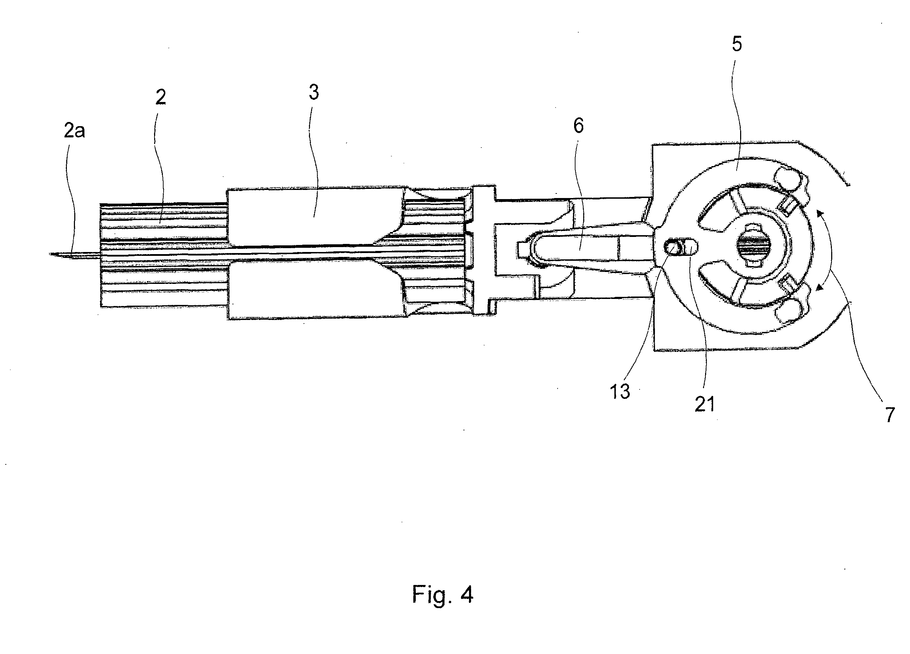

In Fig. 4 ist in einer Draufsicht ein Ausschnitt der erfindungsgemäßen Stechvorrichtung, wie sie in Fig. 1 - 3 wiedergegeben wird, wiedergegeben. Dieser Darstellung ist zu entnehmen, dass das Drehelement 5 bei einer Drehbewegung 7 die Pleuelstange 6 mit dem Zapfen 13 innerhalb eines Langloches 21 bewegt. Dies ermöglicht den nötigen Spielraum bei der Umsetzung der Drehbewegung 7 des Drehelementes 5 in die Schwenkbewegung der Pleulstange 6 und der sich anschließenden Linearbewegung des Nadelhalteelementes 3.

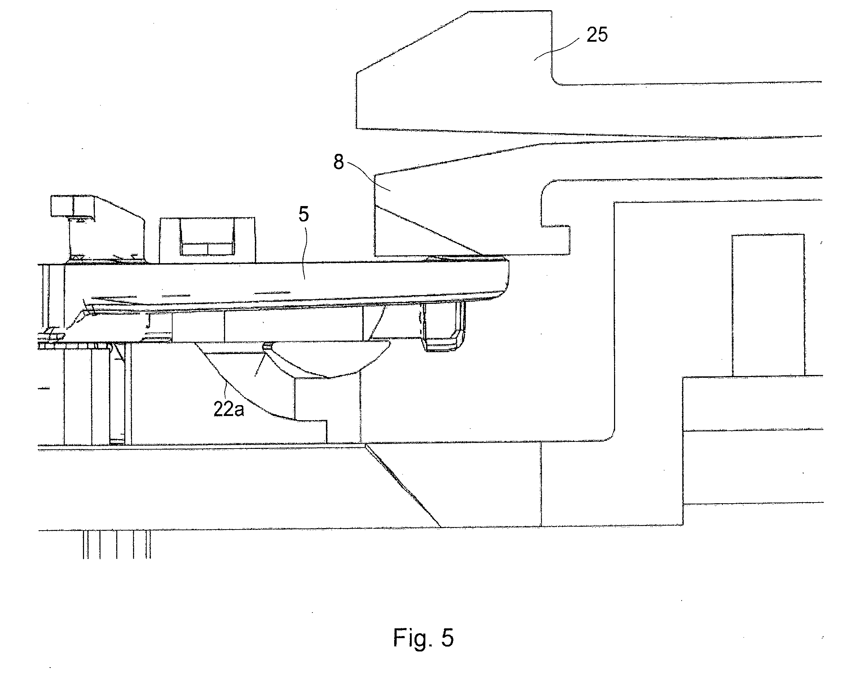

In Fig. 5 ist in einer Seitendarstellung ein Ausschnitt mit dem Drehelement 5 gemäß der erfindungsgemäßen Stechvorrichtung wiedergegeben. Dieser Darstellung ist zu entnehmen, dass bei Drehung des Drehelementes 5 das auf dieser Seite angeordnete zungenartige Element 5a entlang des Abschnitts 22a der Kurvenbahn, die rampenartig ausgebildet ist, nach unten gezogen werden kann, um den Vorsprung 10 an dem Eingreifarm 8 unterhalb vorbeigleiten zu lassen und somit nicht hindern eine Eingreifposition zwischen dem Eingreifarm 8 und dem Vorsprung 10 stattfinden zu lassen, da indessen eine Eingreifposition zwischen dem Eingreifarm 9 und dem Vorsprung 11 momentan stattfinden soll.

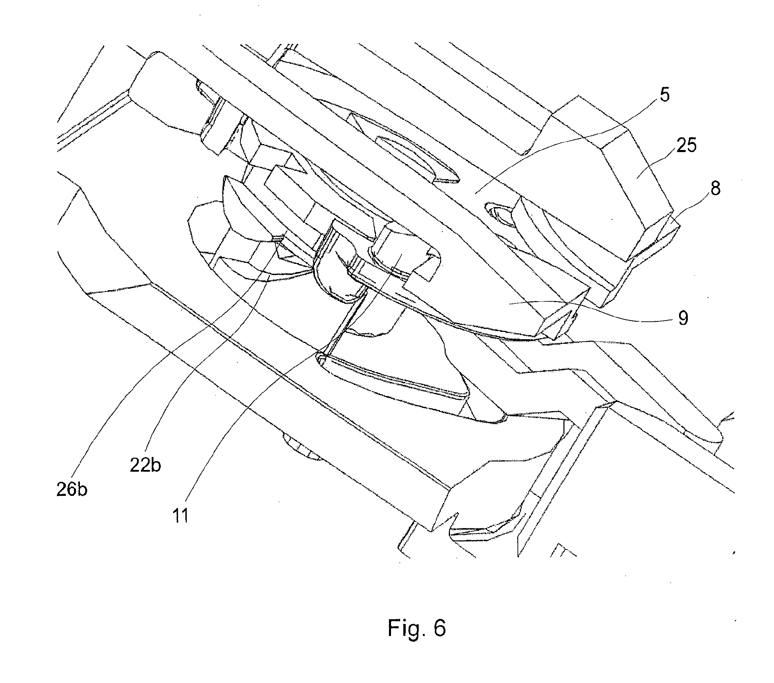

In Fig. 6 ist in einer perspektivischen Darstellung nochmal der Ausschnitt mit dem Drehelement und den Eingreifarmen gemäß der erfindungsgemäßen Stechvorrichtung wiedergegeben. Deutlich ist der Darstellung zu entnehmen, dass auf der anderen Seite, also auf der Seite des Eingreifarmes 9, ebenso ein rampenartiger Abschnitt 22b der Kulissenbahn beziehungsweise Kurvenbahn angeordnet ist, entlang welcher ebenso der Vorsprung 11 bei nicht stattfindender Eingreifsituation zwischen dem Eingreifarm 9 und dem Vorsprung 11 unterhalb des Eingreifarmes 9 entlanggleiten kann und somit der Eingreifarm 9 nicht zum Eingreifen in den Vorsprung 11 gelangt.

In der momentanen Position des Drehelementes 5 ist in der Darstellung gemäß Fig. 6 jedoch ein Eingreifen zwischen dem Vorsprung 11 und dem Eingreifarm 9 vorhanden und der Vorsprung 11 kann in entlang eines weiteren Abschnitts 26b der Kurvenbahn in Eingreifposition oberhalb des Abschnitts 22b entlanggleiten und bleibt hierbei in Position zu dem Eingreifarm 9. Auf diese Weise kann immer einer der beiden Vorsprünge 10, 11 entlang des Abschnitts 22a oder 22b nach unten verschoben werden, während der andere der beiden Vorsprünge 10, 11 mittels einer der beiden Eingreifarme 8 oder 9 in Eingreifposition nach hinten gezogen wird. Dies hat zur Folge, dass das Drehelement sich den Zapfen 13 einer Position - bezogen auf die Längsachse 20 - im linken Bereich in eine Position in den rechten Bereich bewegt oder vice versa.

Sofern es sich um eine nach rechts gerichtete Drehbewegung des Drehelements handelt, wird der Vorsprung 10 von dem Eingreifarm 8 gezogen. Wenn es sich um eine linksgerichtete Drehbewegung handelt, wird der Vorsprung 11 von rechts nach links und nach hinten mittels des Eingreifarmes 9 gezogen. In beiden Fällen findet jeweils ein Aus- und Einfahren der Nadel 2 und ein Vorwärts- und Rückwärts-Verfahren des Nadelhalteelementes 3 statt, da das erste Ende 14a der Pleuelstange 6 mittels des Zapfens 13 ein Maximum, welches in Richtung des Stechens gerichtet ist, läuft, während die Drehbewegung stattfindet. Vorteilhaft wird hierdurch aufgrund einer einzigen Drehbewegung ein Ein- und Ausfahren des Nadelhalteelementes während des Stechvorganges erreicht.

Anschließend kann wiederum durch eine entgegengesetzte Drehbewegung ein Ein- und Ausfahren für einen weiteren Stechvorgang durchgeführt werden. Zwischen den beiden Stechvorgängen kann jedoch mittels des Eingreifarmes 25 eine Vorspannung der Spiralfeder 25 erfolgen, ohne dass hierdurch das Drehelement und somit auch das Nadelhalteelement 3b bewegt werden müssen.

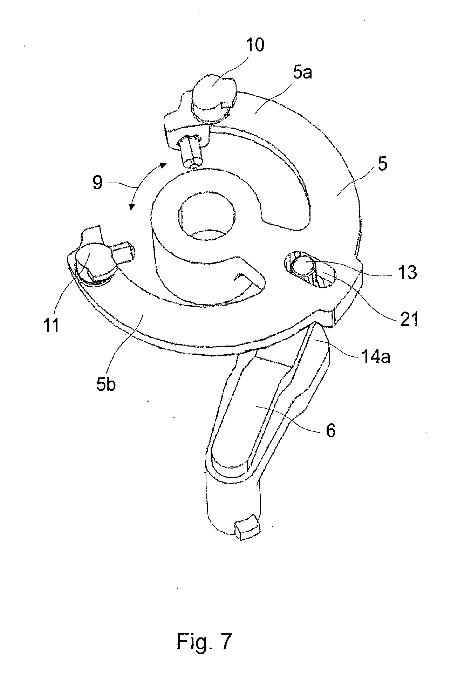

In Fig. 7 ist in einer perspektivischen Darstellung nochmals das Drehelement 5 mit den beiden zungenartigen Elementen 5a und 5b, auf denen die Vorsprünge 10, 11 angeordnet sind, wiedergegeben. Dieser Darstellung ist deutlich zu entnehmen, dass der Zapfen 13 des ersten Endes 14a der Pleuelstange 6 in ein Langloch 21 eingreift, welches das notwendige Zusammenspiel von Pleuelstange und Drehelement 5 zur Verfügung stellt.

Die Vorsprünge 10, 11 sind derart ausgebildet, dass sie vorteilhaft zungenartige Vorsprünge an der Stelle aufweisen, wo die Eingreifarme 8, 9 clipsartig eingreifen können.

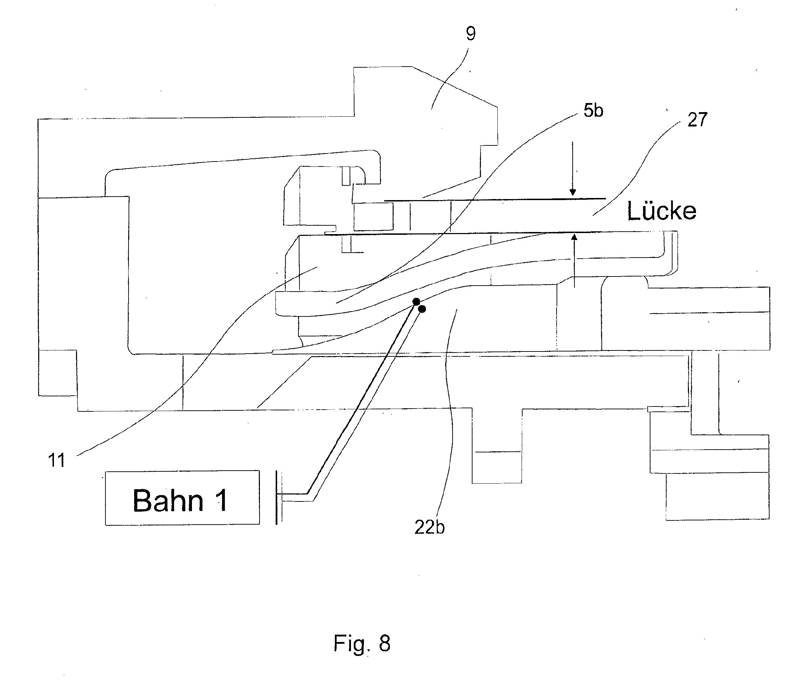

In Fig. 8 ist das Drehelement zusammen mit der Kurvenbahn gemäß einer ersten Ausführungsform der Erfindung gezeigt. In dieser Darstellung ist zusehen, dass die Kurvenbahn 22b rampenartig ausgebildet sein kann und der Vorsprung 11 zusammen mit dem zungenartigen Element 5b sich gerade in der abgesenkten Form aufgrund des Entlanglaufens der rampenartigen Kurvenbahn 22b befindet. In dieser Position kann der Eingreifarm 9 über den Vorsprungarm 11 hinweggleiten, ohne diesen zu berühren. Dies ist in dieser Position erwünscht, in der momentan der Eingreifarm 8 in den Vorsprung 10 eingreifen soll. Eine Lücke 27 ist somit zwischen dem Eingreifarm 9 und dem Vorsprung 11 vorhanden.

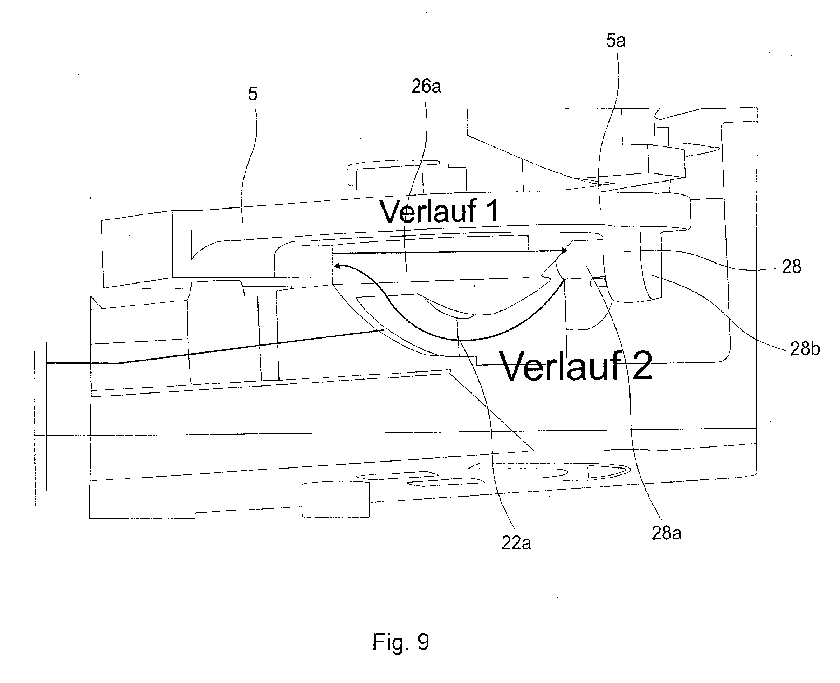

In Fig. 9 ist in einem Ausschnitt das Drehelement zusammen mit einer Kurvenbahn gemäß einer zweiten Ausführungsform der Erfindung wiedergegeben. Dieser Darstellung ist zu entnehmen, dass die Kurvenbahn den ersten Abschnitt 22a und den zweiten Abschnitt 26a aufweist. Somit kann das zungenartige Element 5a mit dem unterseitig angeordneten Vorsprung 28 bei einer linksgerichteten Drehbewegung des Drehelements unterhalb des Eingreifarmes hindurchlaufen, wie es durch den Kurvenbahnabschnitt 22a wiedergegeben wird. Wenn hingegen eine rechtsgerichtete Drehbewegung des Drehelementes 5 stattfinden soll, verläuft der Vorsprung 28 und damit das zungenartige Element 5a auf einem oberen Kurvenbahnabschnitt 26a, um mit dem Eingreifarm 8 in Eingreifposition zu verbleiben.

Sichergestellt wird diese Funktionsweise der verschiedenen Läufe bei einer Rechts- und Linksdrehung durch die Ausbildung des Vorsprunges 28, der mittels eines kleineren zylinderartigen Elementes 28a in den Kurvenbahnabschnitt 26a verläuft und mittels eines größeren zylinderartigen Abschnitts 28b in dem unteren Kurvenbahnabschnitt 22a verläuft.

Durch eine derartige Ausbildung des Drehelementes, welches nach links und rechts gedreht werden kann und ein Zusammenspiel mit den Eingreifarmen 8, 9 sowie insbesondere dem Kurvenbahnabschnitt 22a, 22b und gegebenenfalls 26a und 26b wird ermöglicht, dass die Eingreifarme 8, 9 abwechselnd in einen der Vorsprünge 10, 11 eingreifen können, um die Nadel in Vorwärts- und Rückwärtsrichtung während eines Stechvorganges zu bewegen und nach erfolgtem Stechvorgang ein Vorspannen der Antriebseinheit von Hand möglich ist, ohne dass hierfür die Nadel bewegt werden muss. Vielmehr verbleibt die Nadel in Ruheposition des Vorspannvorganges und anschließend kann der andere Vorsprung mit dem anderen Eingreifarm zur Durchführung des weiteren Stechvorganges verwendet werden.

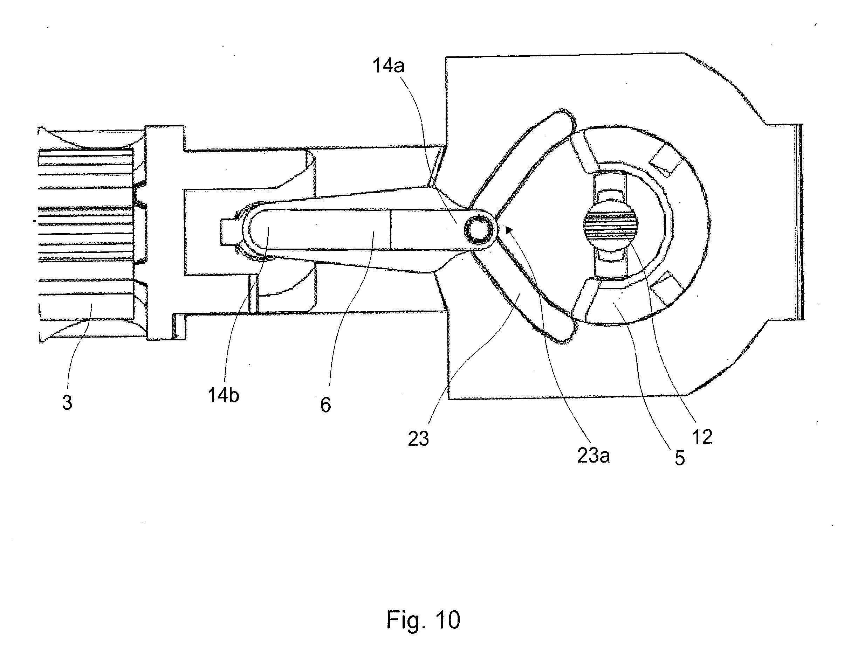

In Fig. 10 wird eine weitere Kurvenbahn 23 gezeigt, in der die Pleuelstange 6 ebenso eingreift.

### Bezugszeichenliste

- 1: Antriebseinheit
- 2: Nadel
- 2a: Spitzende
- 3: Nadelhalteelement
- 5: Drehelement
- 5a: erstes zungenartiges Element
- 5b: zweites zungenartiges Element
- 6: Pleuelelement
- 7: Drehbewegung
- 8: Eingreifarm
- 9: Eingreifarm
- 10: Vorsprung
- 11: Vorsprung
- 12: Drehachse
- 13: Vorsprung
- 14a: erstes Ende des Pleuelelements
- 14b: zweites Ende des Pleuelelements
- 15: Vorsprung
- 16: Basiskörper
- 17: Bewegung in Rückwärtsrichtung
- 18: Linearbewegung
- 19: Stechrichtung
- 20: Längsachse
- 21: Langloch
- 22a: ersten Abschnitt der Kurvenbahn
- 22b: zweiter Abschnitt der Kurvenbahn
- 23: weitere Kurvenbahn
- 23a: Maximum
- 24: Spiralfeder
- 25: Eingreifarm
- 26a: Kurvenbahnabschnitt
- 26b: Kurvenbahnabschnitt
- 27: Lücke zwischen Engreifarm und Vorsprung
- 28: Vorsprung
- 28a: zylinderartiges Element

## Patentansprüche

1. Stechvorrichtung für die Blutentnahme bei medizinischen Untersuchungen mit einem Basiskörper (16), mindestens einer darin angeordneten und mit einem Spitzende (2a) herhausfahrbaren Nadel (2), mit einem die Nadel (2) zumindest teilweise umfassenden Nadelhalteelement (3) und mit einer Antriebseinheit (1) zum Antreiben einer Bewegung (17) der Nadel (2) zusammen mit dem Nadelhalteelement (3) gegenüber dem Basiskörper (16), wobei ein Drehelement (5) zur Durchführung einer Drehbewegung (7) zwischen der Antriebseinheit (1) und dem Nadelhalteelement (3) als Verbindungselement angeordnet ist, und die Drehbewegung (7) sowohl links- als auch rechtsgerichtet jeweils die Bewegung (17) des Nadelhalteelementes (3) sowohl in Vorwärts- als auch in Rückwärtsrichtung (17) bewirkt.

2. Stechvorrichtung nach Anspruch 1,
**dadurch gekennzeichnet, dass**
das Antriebselement (1) linear beweglich ist (18) und mindestens zwei Eingreifarme (8, 9) aufweist, wovon bei jeder Drehbewegung (7) jeweils einer (9) in einen (11) von mindestens zwei Vorsprüngen (10, 11) des Drehelementes (5) eingreift und der andere Vorsprung (10) mittels einer Kurvenbahn (22a, 22b) an dem anderen Eingreifarm (8) vorbeilenkbar ist, ohne dass er (10) mit dem anderen Eingreifarm (8) eine Eingreifposition einnimmt.

3. Stechvorrichtung nach Anspruch 1 oder 2,
**dadurch gekennzeichnet, dass**
der erste Vorsprung (10) und der erste Eingreifarm (8), in Stechrichtung (19) betrachtet, links von einer Längsachse (20), die durch eine Drehachse (12) des Drehelementes (5) verläuft, und der zweite Vorsprung (11) sowie der zweite Eingreifarm (9) rechts von der Längsachse (20) angeordnet sind.

4. Stechvorrichtung nach Anspruch 2 oder 3,
**dadurch gekennzeichnet, dass**
das Drehelement als scheibenartiges Element (5) mit der senkrecht zur Scheibenebene und zur Richtung der Bewegung (17) des Nadelhalteelementes (3) angeordneten Drehachse (12) ausgebildet ist und die beiden Vorsprünge (10, 11) auf biegsam ausgebildete, zungenartige Elemente (5a, 5b) der Scheibenebene randseitig angeordnet sind, wobei die zungenartigen Elemente (5a, 5b) in Richtung des Verlaufs der Drehachse (12) auslenkbar sind.

5. Stechvorrichtung nach Anspruch 4,
**dadurch gekennzeichnet, dass**
ein erster Abschnitt (22a) der Kurvenbahn unterhalb eines ersten zungenartigen Elementes (5a) und ein zweiter Abschnitt (22b) unterhalb eines zweiten zungenartigen Elementes (5b) mit einer Steigung zu dem scheibenartigen Element (5) hin angeordnet sind.

6. Stechvorrichtung nach Anspruch 5,
**dadurch gekennzeichnet, dass**
die Abschnitte (22a, 22b) der Kurvenbahn kreisbogenförmig ausgebildet sind.

7. Stechvorrichtung nach einem der vorangegangenen Ansprüche,
**gekennzeichnet durch**
mindestens ein Pleuelelement (6), welches das Drehelement (5) mit dem Nadelhalteelement (3) gelenkig verbindet, wobei ein erstes Ende (14a) des Pleuelelements (6) mit einem Vorsprung (13) in ein am Drehelement (5) angeordnetes Langloch (21) eingreift und ein zweites Ende (14b) des Pleuelelementes (6) mit einem Vorsprung (15) in eine komplementär dazu ausgebildete Ausnehmung des Nadelhalteelementes (3) eingreift.

8. Stechvorrichtung nach Anspruch 7,
**dadurch gekennzeichnet,dass**
das erste Ende (14a) des Pleuelelementes (6) ebenso in eine mit dem Basiskörper (16) verbundene weitere Kurvenbahn (23) eingreift, deren Verlauf V-förmig und/oder kurvenförmig mit einem mittig angeordneten Maximum (23a) in Richtung (19) des Nadelhalteelementes (3) ausgestattet ist.

9. Stechvorrichtung nach einem der vorangegangenen Ansprüche,
**dadurch gekennzeichnet, dass**
die Antriebseinheit (1) mit mindestens einem Federelement, vorzugsweise einer in Stechrichtung (19) wirkenden Spiralfeder (24), federbeaufschlagbar ist.

10. Stechvorrichtung nach einem der Ansprüche 3 - 9,
**dadurch gekennzeichnet, dass**
während einer Drehbewegung (7) des Drehelementes (5) jeweils ein Vorsprung (10, 11) durch dem darin eingreifenden Eingreifarm (8, 9) auf einer Kreisbogenbahn in einer Ebene von der einen Seite auf die andere Seite der Längsachse (20) bewegbar ist.

## Claims

1. Lancing device for taking blood for medical analyses, comprising a base body (16), at least one needle (2) which is arranged therein and can be extended by a tip (2a), comprising a needle holding element (3) which encloses the needle (2) at least in part, and comprising a drive unit (1) for driving a movement (17) of the needle (2) together with the needle holding element (3) with respect to the base body (16), wherein a rotary element (5) for carrying out a rotary movement (7) is arranged between the drive unit (1) and the needle holding element (3) as a connection element, and the rotary movement (7), both left and right, in each case brings about the movement (17) of the needle holding element (3) both forwards and backwards (17).

2. Lancing device according to claim 1, **characterised in that** the drive element (1) can be moved linearly (18) and has at least two engagement arms (8, 9), one (9) of which in each case engages in one (11) of at least two projections (10, 11) on the rotary element (5) and the other projection (10) is deflectable on the other engagement arm (8) by means of a curved path (22a, 22b), without said projection (10) taking on an engagement position with the other engagement arm (8), during each rotary movement (7).

3. Lancing device according to claim 1 or claim 2, **characterised in that** the first projection (10) and the first engagement arm (8), viewed in the lancing direction (19), are arranged to the left of a longitudinal axis (20) which extends through an axis of rotation (12) of the rotary element (5), and the second projection (11) and second engagement arm (9) are arranged to the right of the longitudinal axis (20).

4. Lancing device according to claim 2 or claim 3, **characterised in that** the rotary element is in the form of a disc-like element (5) having the axis of rotation (12) arranged perpendicularly to the plane of the disc and to the direction of movement (17) of the needle holding element (3), and the two projections (10, 11) are arranged at the edges of flexibly formed tongue-like elements (5a, 5b) of the disc plane, the tongue-like elements (5a, 5b) being deflectable in the direction of the extension of the axis of rotation (12).

5. Lancing device according to claim 4, **characterised in that** a first portion (22a) of the curved path is arranged below a first tongue-like element (5a) and a second portion (22b) is arranged below a second tongue-like element (5b) inclined towards the disc-like element (5).

6. Lancing device according to claim 5, **characterised in that** the portions (22a, 22b) of the curved path are formed in an arc shape.

7. Lancing device according to any of the preceding claims, **characterised by** at least one connecting rod element (6) which articulates the rotary element (5) to the needle holding element (3), a first end (14a) of the connecting rod element (6) engaging, by means of a projection (13), in a slot (21) arranged in the rotary element (5) and a second end (14b) of the connecting rod element (6) engaging, by means of a projection (15), in a recess formed complementarily thereto in the needle holding element (3).

8. Lancing device according to claim 7, **characterised in that** the first end (14a) of the connecting rod element (6) also engages in a further curved path (23) connected to the base body (16), the curve profile of which is equipped, in a V-shape and/or curved shape, with a centrally arranged maximum (23a) in the direction (19) of the needle holding element (3).

9. Lancing device according to any of the preceding claims, **characterised in that** the drive unit (1) is spring-loadable by means of at least one spring element, preferably a spiral spring (24) acting in the Lancing direction (19).

10. Lancing device according to any of claims 3-9, **characterised in that**, during a rotary movement (7) of the rotary element (5), in each case one projection (10, 11) can be moved, by the engagement arm (8, 9) engaging therein, from one side to the other side of the longitudinal axis (20) on an arced path in one plane.

## Revendications

1. Dispositif de piquage pour le prélèvement de sang lors d'examens médicaux avec un corps de base (16), au moins une aiguille (2) rétractable disposée à l'intérieur et avec une extrémité pointue (2a), avec un élément de maintien de l'aiguille (3) entourant au moins partiellement l'aiguille (2) et avec une unité d'entraînement (1) pour l'entraînement d'un mouvement (17) de l'aiguille (2) en même temps que l'élément de maintien de l'aiguille (3) vis-à-vis du corps de base (16), un élément rotatif (5) étant disposé pour la réalisation d'un mouvement de rotation (7) entre l'unité d'entraînement (1) et l'élément de maintien de l'aiguille (3) en tant qu'élément de liaison et le mouvement de rotation (7), orienté aussi bien à gauche qu'à droite, provoque respectivement le mouvement (17) de l'élément de maintien de l'aiguille (3) aussi bien vers l'avant que vers l'arrière (17).

2. Dispositif de piquage selon la revendication 1, **caractérisé en ce que** l'élément d'entraînement (1) est mobile linéairement (18) et comprend au moins deux bras d'emboîtement (8, 9), dont, à chaque mouvement rotatif (7), un bras (9) s'emboîte dans une saillie (11) parmi au moins deux saillies (10, 11) de l'élément rotatif (5) et l'autre saillie (10) peut être dirigé à l'aide d'une piste incurvée (22a, 22b) le long de l'autre bras d'emboîtement (8), sans qu'elle (10) adopte une position d'emboîtement avec l'autre bras d'emboîtement (8).

3. Dispositif de piquage selon la revendication 1 ou 2, **caractérisé en ce que** la première saillie (10) et le premier bras d'emboîtement (8), vus dans la direction de piquage (19), sont disposés à gauche d'un axe longitudinal (20), qui traverse un axe de rotation (12) de l'élément rotatif (5), et la deuxième saillie (11) et que le deuxième bras d'emboîtement (9) sont disposés à droite de l'axe longitudinal (20).

4. Dispositif de piquage selon la revendication 2 ou 3, **caractérisé en ce que** l'élément rotatif est conçu comme un élément en forme de disque (5) avec l'axe de rotation (12) disposé perpendiculairement au plan du disque et à la direction du mouvement (17) de l'élément de maintien de l'aiguille (3) et les deux saillies (10, 11) sont disposées sur des éléments en forme de languettes (5a, 5b) flexibles, au bord du plan du disque, les éléments en forme de languettes (5a, 5b) pouvant être dirigés dans la direction du cours de l'axe de rotation (12).

5. Dispositif de piquage selon la revendication 4, caractérisé au ce qu'une première portion (22a) de la piste incurvée est disposée en dessous d'un premier élément en forme de languette (5a) et une deuxième portion (22b) est disposée au dessous d'un deuxième élément en forme de languette (5b) avec une pente en direction de l'élément en forme de disque (5).

6. Dispositif de piquage selon la revendication 5, **caractérisé en ce que** les portions (22a, 22b) de la piste incurvée présentent une forme d'arc de cercle.

7. Dispositif de piquage selon l'une des revendications précédentes, **caractérisé par** au moins un élément de bielle (6) qui relie de manière articulée l'élément rotatif (5) avec l'élément de maintien de l'aiguille (3), une première extrémité (14a) de l'élément de bielle (6) s'emboîte avec une saillie (13) dans un trou oblong (21) disposé sur l'élément rotatif (5) et une deuxième extrémité (14b) de l'élément de bielle (6) s'emboîte avec une saillie (15) dans un évidement complémentaire de l'élément de maintien de l'aiguille (3).

8. Dispositif de piquage selon la revendication 7, **caractérisé en ce que** la première extrémité (14a) de l'élément de bielle (6) s'emboîte également dans une autre piste incurvée (23) reliée au corps de base (16), dont le trajet est muni, sous une forme de V ou courbée, d'un maximum (23a) disposé au centre en direction (19) de l'élément de maintien de l'aiguille (3).

9. Dispositif de piquage selon l'une des revendications précédentes, **caractérisé en ce que** l'unité d'entraînement (1) peut être contrainte par au moins un élément à ressort, de préférence un ressort à spirale (24) agissant dans la direction de piquage (19).

10. Dispositif de piquage selon l'une des revendications 3 à 9, **caractérisé en ce que**, pendant un mouvement rotatif (7) de l'élément rotatif (5), à chaque fois une saillie (10, 11) est mobile à travers le bras d'emboîtement (8, 9) qui s'y emboîte, sur une piste en arc de cercle dans un plan d'un côté vers l'autre côté de l'axe longitudinal (20).
